# EUROPÄISCHE PATENTSCHRIFT

(11) **EP 1 462 433 B1**
(45) Veröffentlichungstag und Bekanntmachung des Hinweises auf die Patenterteilung: **23.10.2013**
(21) Anmeldenummer: 04100261.9
(22) Anmeldetag: 26.01.2004
(51) Int. Cl.: C07C 29/80, C07C 31/12

(54) **Verfahren zur Abtrennung von 2-Butanol aus tert.-Butanol/Wasser-Gemischen**
Process for the separation of 2-butanol from tert.-butanol/water mixtures
Procédé pour la séparation de 2-butanol d' un mélange tert.-butanol/eau

(30) Priorität: 22.03.2003 DE 10312918
(43) Veröffentlichungstag der Anmeldung: 29.09.2004
(73) Patentinhaber: Evonik Degussa GmbH, 45128 Essen (DE)
(72) Erfinder: Beckmann, Andreas, Dr., 45657, Recklinghausen (DE); Reusch, Dieter, Dr., 45772, Marl (DE)
(74) Vertreter: Hirsch, Hans-Ludwig

(56) Entgegenhaltungen:
- EP-A1- 0 304 770
- JP-B- 50 016 339
- DATABASE CHEMABS [Online] CHEMICAL ABSTRACTS SERVICE, COLUMBUS, OHIO, US; INASHIMA, MAKOTO ET AL: "Purification of a crude tert-butyl alcohol" XP002279771 gefunden im STN Database accession no. 1975:605762 & JP 500 163 39B B4 (MARUZEN OIL COMPANY, LTD., JAPAN) 12. Juni 1975 (1975-06-12)

## Beschreibung

Die vorliegende Erfindung betrifft ein Verfahren zur Abtrennung von 2-Butanol (im Text auch als sekundäres Butanol oder SBA bezeichnet) aus tert.-Butanol/Wasser-Gemischen, die bei der Spaltung von tert.-Butanol (TBA), insbesondere solchem hergestellt aus technischen C₄-Kohlenwasserstoffgemischen, zu Isobuten und Wasser anfallen.

Eine Abtrennung von SBA aus einem technischen Gemisch, das SBA, TBA und Wasser aufweist, wobei der Massenanteil an Wasser in dem Gemisch sicher größer ist als die Grenzkonzentrationen der beiden Azeotrope TBA/Wasser und SBA/Wasser verbindenden Destillationsgrenzlinie, wobei in einem ersten Schritt aus dem Gemisch mit Hilfe eines Destillationsverfahrens Wasser abgetrennt wird und bei dem in einem zweiten Schritt das Gemisch destillativ in einem SBA aufweisenden Strom und einem überwiegend TBA und Wasser aufweisenden Strom getrennt wird, ist aus EP 0 304 770 A1 bekannt. Isoliert betrachtet, findet es in den in Figur 4 gezeigten Kolonnen 4 und 5 statt.

Bei dem Eingangsstrom 25 des in Figur 4 der EP 0 304 770 A1 gezeigten Prozesses handelt es sich um ein Gemisch aus Wasser, TBA und SBA, was allerdings aufgrund seines dominanten SBA-Anteils nicht dem Oberbegriff des Anspruch 1 entspricht.

Isobuten ist ein Ausgangsstoff für die Herstellung von Butylkautschuk, Polyisobutylen, Isobuten-Oligomeren, verzweigten C₅-Aldehyden und C₅-Carbonsäuren. Weiterhin wird es als Alkylierungsmittel und als Zwischenprodukt zur Erzeugung von Peroxiden eingesetzt.

In technischen Strömen liegt Isobuten zusammen mit gesättigten und ungesättigten C₄-Kohlenwasserstoffen vor. Aus diesen Gemischen kann Isobuten wegen der geringen Siedepunkts-differenz bzw. des sehr geringen Trennfaktors zwischen Isobuten und 1-Buten durch Destilla tion nicht wirtschaftlich abgetrennt werden. Daher wird Isobuten aus technischen Kohlenwassersboffgemischen dadurch gewonnen, dass Isobuten zu einem Derivat umgesetzt wird, das sich leicht vom übrig gebliebenden Kohlenwasserstoffgemisch abtrennen lässt, und dass das isolierbe Derivat zu Isobuten und Derivatisierungsmittel zurückspaltet wird.

Üblicherweise wird Isobuten aus C₄-Schnitten, beispielsweise der C₄-Fraktion eines Steamcrackers, wie folgt abgetrennt. Nach Entfernung des größten Teils der mehrfach ungesättigten Kohlenwasserstoffe, hauptsächlich Butadien, durch Extraktion(-sdestillation) oder Selektivhydrierung zu linearen Butenen wird das verbleibende Gemisch (Raffinat I oder hydriertes Crack-C₄) mit Alkohol oder Wasser umgesetzt. Bei der Verwendung von Methanol als Alkohol entsteht Methyl-tert.-butylether (MTBE) und bei Einsatz von Wasser tert-Butanol (TBA). Nach ihrer Abtrennung können beide Produkte in Umkehrung ihrer Bildung zu Isobuten gespalten werden.

Die Spaltung von TBA lässt sich leichter als die Spaltung von MTBE durchführen und ergibt weniger Nebenprodukte und ist somit das bevorzugte Verfahren zur Gewinnung von Isobuten. Bevorzugt die Spaltung von TBA in Gegenwart einer Säure in der Gas- oder Flüssigphase unter Teilumsatz von TBA durchgeführt.

Werden zur Herstellung von TBA aus Isobuten Isobuten-haltige Kohlenwasserstoffströme, die auch lineare Butene enthalten, eingesetzt, entsteht in geringen Mengen auch 2-Butanol (SBA).

Dies ist solange nicht weiter kritisch, wie das entstandene Reaktionsgemisch in ein reines TBA oder in ein TBA/Wasser-Azeotrop aufgearbeitet wird. Dabei wird wegen des nur geringen 2-Butanolgehalts im Reaktionsgemisch die maximal zulässige 2-Butanolkonzenzentration von beispielsweise 0,2 Massen-% im TBA oder im TBA/Wasser-Azeotrop nicht überschritten.

Wird das technische TBA oder TBA/Wasser-Azeotrop allerdings unter Teilumsatz in Isobuten und Wasser gespalten, so fällt nach Abtrennung des entstandenen Isobutens ein TBA/Wasser-Gemisch an, in dem das 2-Butanol (SBA) angereichert ist. Dieses Gemisch ist ohne eine Abtrennung von 2-Butanol nicht zur Herstellung von marktgängigen TBA oder TBA/Wasser-Azeotrop geeignet. Ebenfalls ist es nicht zweckmäßig, aus diesem Gemisch Isobuten herzustellen, weil mit zunehmendem 2-Butanolgehalt auch die Konzentration von linearen Butenen im Isobuten steigt, wodurch dessen Spezifikation nicht eingehalten werden kann. Daher ist es notwendig, einen Teil des 2-Butanols unter Vermeidung von TBA-Verlusten auszuschleusen.

Aufgabe der vorliegenden Erfindung war es deshalb ein Verfahren bereitzustellen, mit welchem es möglich ist, SBA aus Gemischen, die SBA, TBA und Wasser aufweisen abzutrennen, ohne dass es zu Verlusten an TBA kommt.

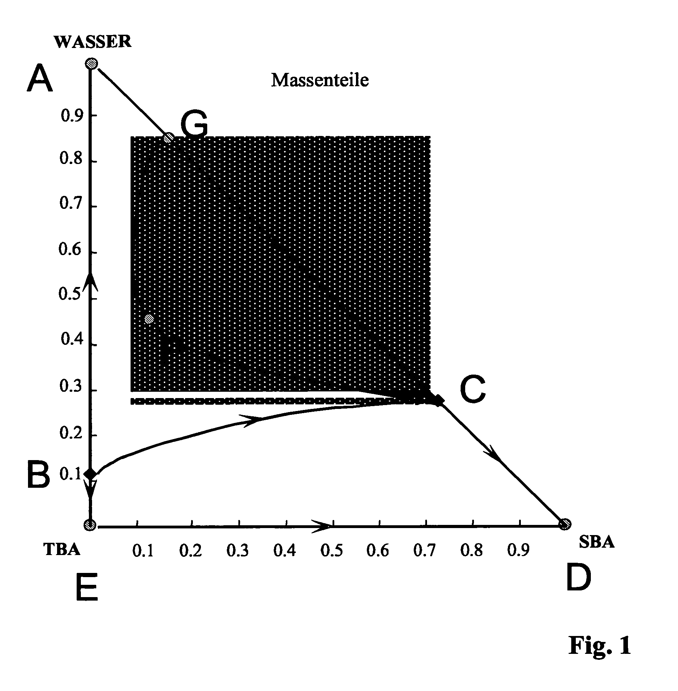

Das Dreistoffgemisch aus SBA, TBA und Wasser ist allerdings destillativ schwierig zu trennen, da in diesem Dreistoffsystem eine Destillationsgrenzlinie (in der Literatur manchmal auch als Grenzdestillationslinie bezeichnet) verläuft, die das binäre Azeotrop Wasser/TBA bei ca. 11 Massen-% Wasser (in der Literatur sind Werte bei Normaldruck von 10 bis 12,5 Massen-% zu finden) (Punkt B in Fig. 1) mit dem binären Azeotrop Wasser/SBA bei ca. 28 Massen-% Wasser (in der Literatur sind Werte bei Normaldruck von 26,7 bis 32 Massen-% zu fmden) (Punkt C in Fig. 1) verbindet. Durch diese Destillationsgrenzlinie werden zwei Destillationsfelder getrennt. Kennzeichnend für das obige Dreistoffsystem, dargestellt in Figur 1, sind zwei Destillationsfelder: Destillationsfeld 1 im Bereich A-B-C-A und Destillationsfeld 2 im Bereich B-E-D-C-B. Im Destillationsfeld 1 findet man als Schwersieder Wasser, der Leichtsieder in diesem Bereich ist das TBA/Wasser-Azeotrop und der Mittelsieder das SBA/Wasser-Azeotrop, das nicht in reiner Form abgetrennt werden kann.

Um SBA aus einem TBA-Isobuten-Anlagenverbund auszuschleusen, ist es am wirtschaftlichsten, den SBA-reichsten Strom dazu zu verwenden. Die bei der TBA-Spaltung erhaltenen Ströme weisen aber einen relativ geringen Gehalt an SBA auf. Üblicherweise weisen sie Zusammensetzungen auf, die im Destillationsfeld 1 liegen. Meistens enthalten diese Ströme auch noch geringe Mengen weiterer Stoffe, deren Gegenwart man in diesem Zusammenhang allerdings nicht zu betrachten braucht. Versucht man ein solches Gemisch mit einer Zusammensetzung im Bereich des Destillationsfeldes 1 destillativ aufzuarbeiten, kann man entweder reines Wasser als Schwersieder und ein Gemisch aus SBA/TBA/Wasser als Kopffraktion gewinnen oder aber im Destillat einer Kolonne als leichtestsiedendes Gemisch das TBA/Wasser-Azeotrop und im Sumpf eine höher siedende Mischung aus SBA/TBA/Wasser mit einem hohen Wasseranteil erhalten. Man kann also aus Massenbilanzgründen und aufgrund des ungünstigen Verlaufs der Destillationslinien in keiner Weise den Gehalt an SBA derart erhöhen, dass eine Ausschleusung dieses Stroms wirtschaftlich sinnvoll ist. Auch die im System vorhandene Mischungslücke (siehe Figur 1: C-F-G-C) kann nicht wirtschaftlich für die Trennung der Komponenten oder deren Anreicherung verwendet werden.

Überraschenderweise wurde aber gefunden, dass aus einem Produktionsstrom, der Wasser, SBA und TBA aufweist und dessen Zusammensetzung im Bereich des Destillationsfelds 1 liegt, insbesondere aus einem Produktionsstrom, in dem SBA angereichert ist, SBA praktisch ohne Verluste an TBA abgetrennt werden kann, wenn dem als Einsatzgemisch verwendeten Produktionsstrom zuerst in einer Kolonne Wasser entzogen wird und dann soviel TBA zugemischt wird, dass das erhaltene Gemisch eine Zusammensetzung aufweist, die im Bereich des Destillationsfelds 2 liegt und somit das Gemisch destillativ in SBA und in ein TBA/Wasser-Gemisch aufgetrennt werden kann.

Gegenstand der Erfindung ist demnach ein Verfahren zur Abtrennung von SBA aus einem technischen Gemisch, das SBA, TBA und Wasser aufweist, wobei der Massenanteil an Wasser in diesem Gemisch größer ist als die Grenzkonzentrationen der die beiden Azeotrope TBA/Wasser und SBA/Wasser verbindenden Destillationsgrenzlinie, also das Gemisch hinsichtlich seiner SBA/TBA/Wasser-Zusammensetzung im Bereich des Destillationsfelds 1 liegt, welches dadurch gekennzeichnet ist, dass durch Zugabe von TBA die Wasserkonzentration im Gemisch soweit abgesenkt wird, dass erhaltene Gemisch hinsichtlich seiner SBA/TBA/Wasser-Zusammensetzung einen Massenanteil an Wasser aufweist, der kleiner ist als die Grenzkonzentration der die beiden Azeotrope TBA/Wasser und SBA/Wasser verbindenden Destillationsgrenzlinie also hinsichtlich seiner SBA/TBA/Wasser-Zusammensetzung im Bereich des Destillationsfeld 2 liegt, und dass dieses Gemisch destillativ in einen SBA aufweisenden Strom und einen überwiegend TBA und Wasser aufweisenden Strom getrennt wird, und dem technischen Gemisch vor der Zuführung des TBA-haltigen Stroms in einer Kolonne Wasser entzogen wird.

Im erfindungsgemäßen Verfahren erfolgt also die SBA-Abtrennung aus einem Gemisch, welches sich bezüglich des Dreistoffsystems SBA,/TBA/Wasser im Destillationsfeld 1 befindet durch Zugabe eines TBA-haltigen Stroms zu diesem Gemisch, wodurch die Zusammensetzung des Dreistoffsystems in das Destillationsfeld 2 verschoben wird, und anschließende Destillation.

Durch das erfindungsgemäße Verfahren ist es nun möglich SBA aus Gemischen abzutrennen, die SBA, TBA und Wasser aufweisen, wobei der Massenanteil an Wasser in diesen Gemischen größer ist als die Grenzkonzentrationen der die beiden Azeotrope TBA/Wasser und SBA/Wasser verbindenden Destillationsgrenzlinie, und damit rein destillativ nicht trennbar sind. Durch die Verwendung eines TBA-haltigen Stroms, der vorzugsweise weniger als 12 Massen-% Wasser sowie eventuell geringe Mengen an Hochsieder (wie z.B. aus Oligomerisierung von Isobuten hervorgegangene C₈- oder C₁₂-Kohlenwasserstoffe, C₈-Alkohole) und/oder geringen Mengen Leichtsieder (wie z.B. Isobuten oder andere C₄-Kohlenwasserstoffe) aufweist zur Veränderung der Konzentration im Gemisch kann auf die Verwendung von Schleppmitteln oder anderen Fremdstoffen verzichtet werden, so dass eine aufwändige Abtrennung dieser Hilfsstoffe vermieden werden kann und die Gefahr der Verunreinigung der Produkte durch diese Hilfsstoffe bei der Aufarbeitung ausgeschlossen wird. Dieser TBA-Strom kann bevorzugt aus einer Anlage zur Herstellung von wasserfreiem TBA gewonnen werden.

Das erfindungsgemäße Verfahren wird nachfolgend beschrieben, ohne dass das Verfahren auf diese Ausführungsformen beschränkt sein soll.

Das erfindungsgemäße Verfahren zur Abtrennung von 2-Butanol (SBA) aus einem technischen Gemisch, das 2-Butanol, tert.-Butanol (TBA) und Wasser aufweist, wobei der Massenanteil an Wasser in dem Gemisch größer ist als die Grenzkonzentrationen der die beiden Azeotrope TBA/Wasser und SBA/Wasser verbindenden Destillationsgrenzlinie, also das Gemisch hinsichtlich seiner SBA/TBA/Wasser-Zusammensetzung im Bereich des Destillationsfelds 1 liegt, zeichnet sich dadurch aus, dass die Wasserkonzentration des Gemisches durch Wasserentzug gefolgt von der Zugabe eines TBA-haltigen Stroms soweit abgesenkt wird, dass das erhaltene Gemisch hinsichtlich seiner SBA/TBA/Wasser-Zusammensetzung einen Massenanteil an Wasser aufweist, der kleiner ist als die Grenzkonzentration der die beiden Azeotrope TBA/Wasser und SBA/Wasser verbindenden Destillationsgrenzlinie, also hinsichtlich seiner SBA/TBA/Wasser-Zusammensetzung im Bereich des Destillationsfeld 2 liegt, und dass dieses Gemisch destillativ in einen SBA aufweisenden Strom und einen TBA und Wasser aufweisenden Strom getrennt wird.

Vorzugsweise wird ein TBA-haltiger Strom zugemischt, der einen Wassergehalt von kleiner 12 Massen-%, bevorzugt kleiner 10 Massen-% und besonders bevorzugt kleiner 5 Massen-% aufweist. Der TBA-haltige Strom kann von 90 bis 99,99 % TBA aufweisen. Neben TBA und gegebenenfalls Wasser kann der TBA-haltige Strom außerdem eventuell geringe Mengen (von 0,0001 bis 5 Massen-%) an Hochsieder (wie z.B. aus Oligomerisierung von Isobuten hervorgegangene C₈- oder C₁₂-Kohlenwasserstoffe, C₈-Alkohole) und/oder Leichtsieder (wie z.B. Isobuten oder andere C₄-Kohlenwasserstoffe) aufweisen.

Dem technischen Gemisch wird vor der Zugabe des TBA-haltigen Stroms destillativ Wasser entzogen. Durch Destillation des technischen Gemisches wird dieses in ein wasserreiches Sumpfprodukt und ein Kopfprodukt aufgetrennt, welches einen Wassergehalt aufweist, der zwar größer ist als die Grenzkonzentration der die beiden Azeotrope TBA/Wasser und SBA/Wasser verbindenden Destillationsgrenzlinie, also hinsichtlich seiner SBA/TBA/Wasser-Zusammensetzung im Bereich des Destillationsfeld 1 liegt, aber geringer als die ursprüngliche Konzentration an Wasser ist. Das so vorbehandelte technische Gemisch wird dann erfindungsgemäß durch Zugabe eines TBA-haltigen Stroms erfindungsgemäß aufgearbeitet. Diese Vorgehensweise hat den Vorteil, dass nur eine relativ geringe Menge an TBA-haltigem Strom zugegeben werden muss, um die Konzentration des Gemisches so zu verändern, dass diese im Bereich des Destillationsfeldes 2 liegt.

Das Gemisch, welchem ein TBA-haltiger Strom zugegeben wurde, und das destillativ aufgearbeitet wird, weist vorzugsweise einen Wassergehalt von kleiner 10 Massen-% bezogen auf das Dreikomponentensystem SBA/TBA/Wasser auf. Wesentlich ist aber dass das Gemisch hinsichtlich seines Wassergehaltes im Destillationsfeld 2 liegt, also einen Massenanteil an Wasser kleiner als der Wassergehalt eines Gemisches mit einer Zusammensetzung entsprechend der die Azeotrope SBA/Wasser und TBA/Wasser verbindenden Destillationsgrenzlinie B-C aufweist. Vorzugsweise beträgt der Wassergehalt bezogen auf das Dreikomponentensystem SBA/TBA/Wasser bei SBA-Gehalten von 0,0001 bis 6 Massen-% weniger als 11 Massen-%, bevorzugt weniger als 10 Massen-% und besonders bevorzugt weniger als 9,5 Massen-%. Bei SBA-Gehalten von 6,01 bis 15 Massen-% beträgt der Wassergehalt bezogen auf das Dreikomponentensystem SBA/TBA/Wasser bevorzugt weniger als 15 Massen-%, bevorzugt weniger als 14 und besonders bevorzugt weniger als 13 Massen-%. Weiterhin kann das Gemisch bis zu 5 Massen-%, vorzugsweise bis zu 3 Massen-%, ganz besonders bevorzugt von 2,5 bis 0,01 Massen-% an weiteren Stoffen, beispielsweise C₈-Olefinen oder -Alkoholen, aufweisen.

Das erfindungsgemäße Verfahren wird vorzugsweise so durchgeführt, dass das Gemisch, welchem ein TBA-haltiger Strom zugegeben wurde, destillativ in eine 2-Butanol aufweisende Fraktion aufgetrennt wird, die weniger als 1 Massen-%, bevorzugt weniger als 0,5 Massen-% tert.-Butanol aufweist. Das 2-Butanol, das bei der destillativen Aufarbeitung abgetrennt wird, kann aus der Dampfphase eines Verdampfers einer Kolonne oder dampfförmig oder flüssig als Seitenstrom im Abtriebsteil dieser Kolonne entnommen werden.

Das bei der destillativen Auftrennung des Gemisches, dem ein TBA-haltiger Strom zugeführt wurde, als Destillat erhaltene Kopfprodukt, kann zumindest teilweise dem technischen Gemisch, dem ein TBA-haltiger Strom zugegeben wird, zugemischt werden. Je nach Ausgangskonzentration des technischen Gemisches und gegebenenfalls erfolgter destillativer Wasserabtrennung kann es sein, dass das Dreistoffgemisch eine Konzentration in der Nähe der Destillationsgrenzlinie B-C aufweist. Bei einer solchen Zusammensetzung kann es sogar ausreichend sein, dass nur ein geringer TBA-haltiger Strom mit einem Teil, des als Destillat erhaltenen Kopfprodukts der Kolonne dem technischen Gemisch zugegeben wird, da auch auf diese Weise die Zusammensetzung des Gemisches in das Destillationsfeld 2 verschoben werden kann.

Die destillative Auftrennung von bei dem erfindungsgemäßen Verfahren anfallenden Stoffströmen, insbesondere des Gemisches, dem ein TBA-haltiger Strom zugegeben wurde, kann in einer oder mehreren Kolonne(n) mit Einbauten, die aus Böden, rotierenden Einbauten, ungeordneten und/oder geordneten Packungen bestehen, durchgeführt werden. Vorzugsweise erfolgt die destillative Trennung dieses Gemisches in einer einzigen Kolonne.

### Bei den Kolonnenböden können folgende Typen zum Einsatz kommen:

Böden mit Bohrungen oder Schlitzen in der Bodenplatte.
Böden mit Hälsen oder Kaminen, die von Glocken, Kappen oder Hauben überdeckt sind.
Böden mit Bohrungen in der Bodenplatte, die von beweglichen Ventilen überdeckt sind.
Böden mit Sonderkonstruktionen.

In Kolonnen mit rotierenden Einbauten wird der Rücklauf entweder durch rotierende Trichter versprüht oder mit Hilfe eines Rotors als Film auf einer beheizten Rohrwand ausgebreitet.

In dem erfindungsgemäßen Verfahren verwendete Kolonnen können regellose Schüttungen mit verschiedenen Füllkörpern eingesetzt werden. Sie können aus fast allen Werkstoffen - Stahl, Edelstahl, Kupfer, Kohlenstoff, Steingut, Porzellan, Glas, Kunststoffen usw. - und in verschiedenen Formen - Kugeln, Ringen mit glatten oder profilierten Oberflächen, Ringen mit Innenstegen oder Wanddurchbrüchen, Drahtnetzringen, Sattelkörper und Spiralen - bestehen.

Packungen mit regelmäßiger Geometrie können z.B. aus Blechen oder Geweben aus Metall oder Kunststoff bestehen. Beispiele solcher Packungen sind Sulzer Gewebepackungen BX, Sulzer Lamellenpackungen Mellapak aus Metallblech, Hochleistungspackungen wie MellapakPlus, Strukturpackungen von Sulzer (Optiflow), Montz (BSH) und Kühni (Rombopak).

Die Kolonne (2) zur Vorentwässerung des als Edukt eingesetzten technischen Gemisches weist vorzugsweise eine theoretische Trennstufenzahl von 3 bis 50, insbesondere eine von 6 bis 40 auf. Der Zulaufboden hängt von der Zusammensetzung des Gemischs im Destillationsfeld 1 ab. Die Einspeisung des Zulaufs erfolgt bevorzugt auf den, von oben gezählten, 2. bis 55. theoretischen Boden, insbesondere auf den 3. bis 35.

Die zur Trennung des durch Zugabe des TBA-haltigen Stroms erhaltenen Gemisches eingesetzte Kolonne weist vorzugsweise eine theoretische Trennstufenzahl von 5 bis 70, bevorzugt eine theoretische Trennstufenzahl von 10 bis 60 auf. Der Zulaufboden hängt von der Zusammensetzung des Gemisches ab. Es hat sich als vorteilhaft erwiesen, wenn die Einspeisung des Gemisches auf den, von oben gezählten, 2. bis 55. theoretischen Boden, insbesondere auf den 3. bis 35. erfolgt.

Der Betriebsdruck der Kolonnen (2) und (6) beträgt bevorzugt von 0,01 bis 15 bar,abs. (bara). Die beiden Kolonnen zur Abtrennung von Wasser aus dem technischen Gemisch vor der Zugabe des TBA-haltigen Stroms (Vorkolonne) und die Kolonne zur destillativen Trennung des nach Zugabe des TBA-haltigen Stroms erhaltenen Gemisches werden bei gleichen oder unterschiedlichen Drücken betrieben. Bei gleichem Druck werden die Kolonnen vorzugsweise bei 0,5 bis 10 bara, bei unterschiedlichem Druck liegt vorzugsweise der Druck im Bereich von 0,5 bis 10 bara für die Vorkolonne und im Bereich von 0,1 bis 10 bara für die Kolonne (6).

Bei der Destillation des durch Zugabe des TBA-haltigen Stroms erhaltenen Gemisches fällt ein Sumpfprodukt als Strom an, das 2-Butanol und gegebenenfalls Hochsieder enthält. Der Gehalt an TBA darin beträgt vorzugsweise kleiner 2 Massen-%, bevorzugt kleiner 1,7 Massen-%. Als Kopfprodukt wird ein Gemisch aus TBA, Wasser und gegebenenfalls Leichtsieder abgezogen. Der Gehalt an 2-Butanol im Kopfprodukt beträgt vorzugsweise kleiner 4 Massen-%, insbesondere kleiner 3 Massen-%. Im Sumpf der Kolonne lässt sich 2-Butanol ohne oder nahezu ohne Hochsieder gewinnen, indem das 2-Butanol aus der Dampfphase des Verdampfers oder dampfförmig oder flüssig als Seitenstrom im Abtriebsteil der Kolonne abgezogen wird.

Die mit dem erfindungsgemäßen Verfahren erhaltenen aus dem Gemisch abgetrennten TBA-Fraktionen können für die bekannten Zwecke verwandt werden. Beispielsweise können sie als Ausgangsmaterial für die Herstellung von Isobuten dienen. Gegebenenfalls darin enthaltene Leichtsieder können destillativ abgetrennt werden.

Das abgetrennte 2-Butanol kann für die üblichen technischen Anwendungen genutzt werden. So kann es z.B. als Vorstufe für Methyl-ethylketon, als Lösemittel für Lacke und Harze, als Bestandteil von Bremsflüssigkeiten sowie als Bestandteil von Reinigungsmitteln eingesetzt werden. Darüber hinaus fmdet es Verwendung bei der Herstellung von Duftstoffen, Farbstoffen und Benetzungsmitteln.

Mit dem erfindungsgemäßen Verfahren kann 2-Butanol ohne Verluste von TBA aus beliebigen ternären Gemischen aus TBA, SBA und Wasser, die im Destillationsfeld 1 liegen, abgetrennt werden. Dies gelingt selbst dann noch, wenn die Gemische zusätzlich bis zu 5 Massen-% Hochsieder (wie z.B. aus Oligomerisierung von Isobuten hervorgegangene C₈- oder C₁₂-Kohlenwasserstoffe, C₈-Alkohole) und/oder Leichtsieder (wie z.B. Isobuten oder andere C₄-Kohlenwasserstoffe) aufweisen. Mittels des erfindungsgemäßen Verfahrens kann also 2-Butanol, insbesondere 2-Butanol, welches einen Gehalt an tert.-Butanol von vorzugsweise kleiner 1, bevorzugt kleiner 0,5 Massen-% aufweist, hergestellt werden.

Im erfindungsgemäßen Verfahren werden insbesondere TBA-Ströme, in denen 2-Butanol angereichert ist, aus Anlagen, in denen Isobuten aus TBA durch Wasserabspaltung hergestellt wird, eingesetzt. Diese Ströme enthalten meistens C₄-Kohlenwasserstoffe und Folgeprodukte von C₄-Olefinen als weitere Komponenten.

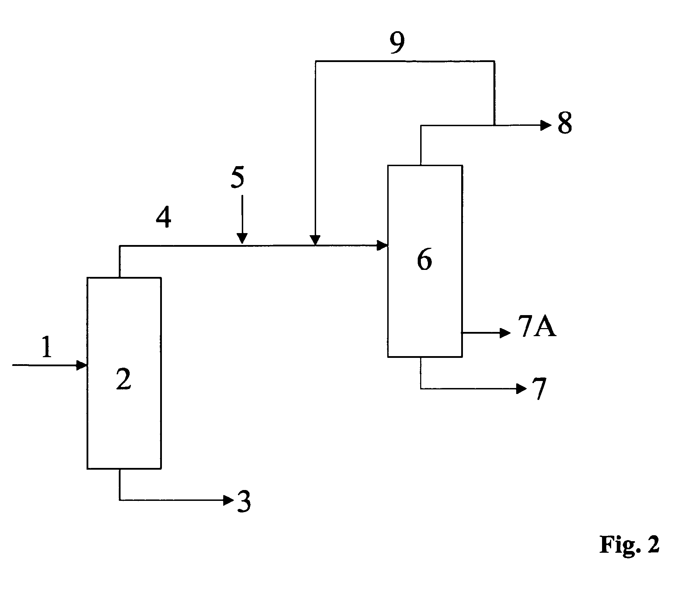

Das Blockschema einer Anlage, in der eine spezielle Ausführungsart des erfindungsgemäße Verfahren durchgeführt werden kann, zeigt die Abbildung in Fig. 2. Das als Edukt dienende technische Gemisch (1), welches eine Zusammensetzung im Destillationsfeld 1 aufweist, wird zunächst in einer Kolonne (2), auch Vorkolonne genannt, derart aufgearbeitet, dass im Sumpf der Vorkolonne ein wasserreicher Strom (3) ausgeschleust wird. Dem auf diese Weise vorentwässerten technischen Gemisch (4), welches immer noch eine Zusammensetzung im Destillationsfeld 1 aufweist, wird nun soviel eines TBA-haltigen Stroms (5) zugegeben, dass das erhaltene Gemisch eine Zusammensetzung aufweist, die im Destillationsfeld 2 liegt. Optional kann diesem Gemisch ein Teil (9) des Destillats (8) aus Kolonne (6) zugegeben werden, wobei durch Zugabe dieses Stroms die Zusammensetzung nicht so verändert werden darf, dass die Zusammensetzung nicht mehr im Destillationsfeld 2 liegt. Dieses Gemisch wird in die Kolonne (6) eingeleitet und in dieser in ein Sumpfprodukt (7) mit dem abzutrennenden 2-Butanol und in ein Kopfprodukt (8), das TBA, Wasser und gegebenenfalls Leichtsieder enthält, getrennt. Der Destillatstrom (8) kann ganz oder teilweise direkt als Zulauf im Anlagenteil einer TBA-Spaltung wieder eingesetzt werden. Optional wird Kolonne (6) bei einem anderen Druck als Kolonne (2) betrieben. Um 2-Butanol mit einem geringen Anteil an Hochsiedern zu gewinnen, kann dieses Produkt aus der Dampfphase des Verdampfers oder dampfförmig oder flüssig als Seitenstrom (7A) im Abtriebsteil der Kolonne (6) abgezogen werden.

Gewöhnliche Bauteile wie Pumpen, Verdichter, Ventile, Wärmetauscher und Verdampfer sind in den Blockschaltbildern nicht dargestellt, jedoch selbstverständliche Bauteile einer Anlage.

Das folgende Beispiel soll die Erfindung erläutern, ohne die Anwendungsbreite einzuschränken, die sich aus der Beschreibung und den Patentansprüchen ergibt.

### Beispiel

Die Abtrennung von SBA erfolgte in einer nach Figur 2 realisierten Anlage, mit der Besonderheit, dass Strom (7A) und (9) entfielen. Der Kolonnendurchmesser der Kolonne (2) betrug dabei 50 mm. Es wurde eine Metall-Destillations-Packung mit 12 theoretischen Stufen installiert, der Zulauf erfolgte auf der, von oben gezählten, 7. theoretischen Stufe. Der Kolonnendurchmesser der Kolonne (6) betrug ebenfalls 50 mm. Es wurde eine Metall-Destillations-Packung mit 20 theoretischen Stufen installiert, der Zulauf erfolgte auf der, von oben gezählten, 6. theoretischen Stufe. Der Zulauf (1) wurde aus der großtechnischen Anlage entnommen und für die Versuche verwendet. Die Stromnummern in der folgenden Tabelle sind die gleichen wie in Figur 2. Das Destillat (4) der Kolonne (2) wurde gesammelt und zum Teil als Zulauf zur zweiten Kolonne (6) eingesetzt. Komponenten mit einer Konzentration kleiner als 0,1 Massen-Teile im Gemisch sind in der Regel nicht in der Tabelle aufgeführt.

| Stromnummer | Strombezeichnung | Massenfluss [kg/h] | Stromzusammensetzung in Massen-Teile |
|---|---|---|---|
| 1 | Frisch-Zulauf | 1,80 | 63,5 Wasser 30,2 TBA 4,5 2-Butanol 1,7 C₈-Alkohol 0,1 sonstige Stoffe |
| 3 | Abwasser | 1,03 | 96,9 Wasser 0,1 TBA 0,1 2-Butanol 2,9 C₈-Alkohol |
| 4 | Vorentwässertes Gemisch, Destillat der Kolonne (2) | 0,77 | 18,9 Wasser 70,3 TBA 10,4 2-Butanol 0,1 C₈-Alkohol 0,3 sonstige Stoffe |
| 4 | Vorentwässertes Gemisch, Zulauf zur Kolonne (6) | 0,75 | 18,9 Wasser 70,3 TBA 10,4 2-Butanol 0,1 C₈-Alkohol 0,3 sonstige Stoffe |
| 5 | Frisch-TBA | 0,75 | 0,004 Wasser 99,8 TBA 0,15 2-Butanol 0,01 C₈-Alkohol 0,036 sonstige Stoffe |
| 7 | Sumpf der Kolonne (6) | 0,08 | 0,3 TBA 96,7 2-Butanol 1,2 C₈-Alkohol 1,9 sonstige Stoffe |
| 7A | Seitenstromentnahme der Kolonne (6) | entfällt | |
| 8 | Destillat der Kolonne (6) | 1,42 | 10,0 Wasser 89,6 TBA 0,4 2-Butanol 0,1 sonstige Stoffe |
| 9 | Recyclestrom | entfällt | |

Die Kolonne (2) wurde bei 1 bara mit einem Rücklaufverhältnis von 3,5 betrieben. Kolonne (6) wurde bei 1 bara mit einem Rücklaufverhältnis von 4 betrieben.

Wie Tabelle 1 zu entnehmen ist, ist mit dem erfindungsgemäßen Verfahren eine Abtrennung von SBA einfach möglich, wobei die Verluste an TBA auf den geringen Anteil im Kopfprodukt der Kolonne beschränkt sind.

## Patentansprüche

1. Verfahren zur Abtrennung von 2-Butanol (SBA) aus einem technischen Gemisch, das 2-Butanol, tert.-Butanol (TBA) und Wasser aufweist, wobei der Massenanteil an Wasser in dem Gemisch größer ist als die Grenzkonzentration der die beiden Azeotrope TBA/Wasser und SBA/Wasser verbindenden Destillationsgrenzlinie,
wobei die Wasserkonzentration des Gemisches durch Zugabe eines TBA-haltigen Stroms soweit abgesenkt wird, dass das erhaltene Gemisch hinsichtlich seiner SBA/TBA/Wasser-Zusammensetzung einen Massenanteil an Wasser aufweist, der kleiner ist als die Grenzkonzentration der die beiden Azeotrope TBA/Wasser und SBA/Wasser verbindenden Destillationsgrenzlinie, und wobei dieses Gemisch destillativ in einen SBA aufweisenden Strom und einen TBA und Wasser aufweisenden Strom getrennt wird,
und wobei dem technischen Gemisch vor der Zuführung des TBA-haltigen Stroms in einer Kolonne Wasser entzogen wird.

2. Verfahren nach Anspruch 1,
**dadurch gekennzeichnet,**
**dass** ein TBA-haltiger Strom zugemischt wird, der einen Wassergehalt von kleiner 12 Massen-% aufweist.

3. Verfahren nach Anspruch 2,
**dadurch gekennzeichnet,**
**dass** ein TBA-haltiger Strom zugemischt wird, der einen Wassergehalt von kleiner 5 Massen-% aufweist.

4. Verfahren nach einem der Ansprüche 1 bis 3,
**dadurch gekennzeichnet,**
**dass** dem technischen Gemisch, dem ein TBA-haltiger Strom zugegeben wird, zusätzlich ein Teil des bei der destillativen Auftrennung dieses Gemisches als Destillat anfallenden Stroms, der Wasser und TBA aufweist, zugemischt wird.

5. Verfahren nach einem der Ansprüche 1 bis 4,
**dadurch gekennzeichnet,**
**dass** das Gemisch, welchem ein TBA-haltiger Strom zugegeben wurde, und der destillativ aufgearbeitet wird, einen Wassergehalt von kleiner 10 Massen-% bezogen auf das Dreikomponentensystem SBA/TBA/Wasser aufweist.

6. Verfahren nach einem der Ansprüche 1 bis 5,
**dadurch gekennzeichnet,**
**dass** das Gemisch, welchem ein TBA-haltiger Strom zugegeben wurde, in eine 2-Butanol aufweisende Fraktion getrennt wird, die weniger als 1 Massen-% tert.-Butanol aufweist.

7. Verfahren nach einem der Ansprüche 1 bis 6,
**dadurch gekennzeichnet,**
**dass** 2-Butanol, das bei der destillativen Aufarbeitung abgetrennt werden, aus der Dampfphase eines Verdampfers einer Kolonne dampfförmig oder flüssig als Seitenstrom im Abtriebsteil dieser Kolonne entnommen wird.

8. Verfahren nach einem der Ansprüche 1 bis 7,
**dadurch gekennzeichnet,**
**dass** die Kolonne zur Abtrennung von Wasser aus dem technischen Gemisch vor der Zugabe des TBA-haltigen Stroms und die Kolonne zur destillativen Trennung des nach Zugabe des TBA-haltigen Stroms erhaltenen Gemisches bei unterschiedlichen Drücken betrieben werden.

## Claims

1. Process for separating 2-butanol (SBA) from an industrial mixture which comprises 2-butanol, tert-butanol (TBA) and water and in which the proportion by mass of water is greater than the limit concentration of the distillation boundary line connecting the two azeotropes TBA/water and SBA/water, wherein the concentration of water in the mixture is reduced by addition of TBA to such an extent that the mixture obtained has, in terms of its SBA/TBA/water composition, a proportion by mass of water which is less than the limit concentration of the distillation boundary line connecting the two azeotropes TBA/water and SBA/water and this mixture is separated by distillation into a stream comprising SBA and a stream comprising TBA and water, and water is removed from the industrial mixture in a column before the TBA-containing stream is fed in.

2. Process according to Claim 1, **characterized in that** a TBA-containing stream which has a water content of less than 12% by mass is added.

3. Process according to Claim 2, **characterized in that** a TBA-containing stream which has a water content of less than 5% by mass is added.

4. Process according to any of Claims 1 to 3, **characterized in that** part of the water- and TBA-containing stream obtained in the fractional distillation of the industrial mixture to which a TBA-containing stream is added is additionally added to this mixture.

5. Process according to any of Claims 1 to 4, **characterized in that** the mixture to which a TBA-containing stream has been added and which is worked up by distillation has a water content of less than 10% by mass, based on the three-component system SBA/TBA/water.

6. Process according to any of Claims 1 to 5, **characterized in that** the mixture to which a TBA-containing stream has been added is separated to give a 2-butanol-containing fraction containing less than 1% by mass of tert-butanol.

7. Process according to any of Claims 1 to 6, **characterized in that** the 2-butanol which is separated off in the work-up by distillation is taken off from the vapor phase of a vaporizer of a column in gaseous or liquid form as side stream in the stripping section of this column.

8. Process according to any of Claims 1 to 7, **characterized in that** the column for separating off water from the industrial mixture prior to the addition of the TBA-containing stream and the column for fractionally distilling the mixture obtained after addition of the TBA-containing stream are operated at different pressures.

## Revendications

1. Procédé de séparation de 2-butanol (SBA) d'un mélange technique comprenant du 2-butanol, du tert.-butanol (TBA) et de l'eau, la proportion en masse d'eau dans le mélange étant supérieure à la concentration seuil de la limite de distillation reliant les deux azéotropes TBA/eau et SBA/eau,
la concentration en eau du mélange étant abaissée par ajout d'un courant contenant du TBA de manière à ce que le mélange obtenu présente au regard de sa composition SBA/TBA/eau une proportion en masse d'eau inférieure à la concentration seuil de la limite de distillation reliant les deux azéotropes TBA/eau et SBA/eau, et ce mélange étant séparé par distillation en un courant comprenant du SBA et un courant comprenant du TBA et de l'eau,
et de l'eau étant extraite du mélange technique dans une colonne avant l'introduction du courant contenant du TBA.

2. Procédé selon la revendication 1, **caractérisé en ce qu'**un courant contenant du TBA présentant une teneur en eau inférieure à 12 % en masse est incorporé.

3. Procédé selon la revendication 2, **caractérisé en ce qu'**un courant contenant du TBA présentant une teneur en eau inférieure à 5 % en masse est incorporé.

4. Procédé selon l'une quelconque des revendications 1 à 3, **caractérisé en ce qu'**une partie du courant comprenant de l'eau et du TBA formé en tant que distillat lors de la séparation par distillation de ce mélange est également incorporée dans le mélange technique auquel un courant contenant du TBA est ajouté.

5. Procédé selon l'une quelconque des revendications 1 à 4, **caractérisé en ce que** le mélange auquel un courant contenant du TBA a été ajouté et qui est traité par distillation présente une teneur en eau inférieure à 10 % en masse par rapport au système tricomposant SBA/TBA/eau.

6. Procédé selon l'une quelconque des revendications 1 à 5, **caractérisé en ce que** le mélange auquel un courant contenant du TBA a été ajouté est séparé en une fraction comprenant du 2-butanol qui comprend moins de 1 % en masse de tert.-butanol.

7. Procédé selon l'une quelconque des revendications 1 à 6, **caractérisé en ce que** le 2-butanol qui est séparé lors du traitement par distillation est soutiré de la phase vapeur d'un évaporateur d'une colonne sous forme vapeur ou liquide en tant que courant latéral dans la zone de rectification de cette colonne.

8. Procédé selon l'une quelconque des revendications 1 à 7, **caractérisé en ce que** la colonne pour la séparation d'eau du mélange technique avant l'ajout du courant contenant du TBA et la colonne pour la séparation par distillation du mélange obtenu après l'ajout du courant contenant du TBA sont utilisées à des pressions différentes.
